(19) Europäisches Patentamt
European Patent Office
Office européen des brevets

(11) Veröffentlichungsnummer: **0 030 616**
**B1**

# (12) EUROPÄISCHE PATENTSCHRIFT

(45) Veröffentlichungstag der Patentschrift: **11.01.84**

(51) Int. Cl.³: **C 07 D 233/50,** A 61 K 31/415

(21) Anmeldenummer: **80106725.7**

(22) Anmeldetag: **03.11.80**

## (54) Neue substituierte 2-Phenylamino-imidazoline-(2), deren Säureadditionssalze, diese enthaltende Arzneimittel und Verfahren zur Herstellung derselben.

(30) Priorität: **07.12.79 DE 2949287**

(43) Veröffentlichungstag der Anmeldung: **24.06.81 Patentblatt 81/25**

(45) Bekanntmachung des Hinweises auf die Patenterteilung: **11.01.84 Patentblatt 84/2**

(84) Benannte Vertragsstaaten: **AT BE CH DE FR GB IT LI LU NL SE**

(56) Entgegenhaltungen:
**AT - B - 285 599**
**DE - A - 1 957 722**
**DE - A - 2 259 160**
**DE - A - 2 523 103**
**DE - A - 2 636 732**
**DE - A - 2 636 732**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim/Rhein (DE)**
Erfinder: **Köppe, Herbert, Dr., Neuweg 72, D-6507 Ingelheim/Rhein (DE)**
Erfinder: **Kummer, Werner, Dr., Georg-Scheuing-Strasse 15, D-6507 Ingelheim/Rhein (DE)**
Erfinder: **Kobinger, Walter, Prof. Dr., Belghofergasse 27, A-1120 Wien (AT)**
Erfinder: **Lillie, Christian, Dr., Hansi-Niese-Weg 12, A-1130 Wien (AT)**
Erfinder: **Pichler, Ludwig, Dr., Gusshausstrasse 24, A-1040 Wien (AT)**
Erfinder: **Hoefke, Wolfgang, Dr., Rosselstrasse 21, D-6200 Wiesbaden (DE)**
Erfinder: **Gaida, Wolfram, Dr., Bahnhofstrasse 74, D-6507 Ingelheim/Rhein (DE)**

**EP 0 030 616 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue substituierte 2-Phenylamino-imidazoline-(2), deren Säureadditionssalze, diese enthaltende Arzneimittel und Verfahren zur Herstellung derselben

Aus DE-A-2 523 103, DE-A-2 636 732, DE-A-1 957 722 und DE-A-2 259 160 sind substituierte 2-Phenylamino-imidazoline-(2) bekannt, denen therapeutische Eigenschaften zugeschrieben werden. Die dort beschriebenen Verbindungen haben insbesondere analgetische, blutdrucksenkende, sedative und sekretionshemmende Eigenschaften.

Erfindungsgemäß werden nun neue substituierte 2-Phenylamino-imidazoline-(2) der allgemeinen Formel (I)

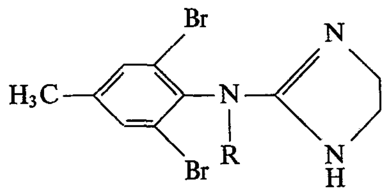 ,

(I)

sowie deren physiologisch verträgliche Säureadditionssalze vorgeschlagen, die gleichfalls wertvolle therapeutische, insbesondere bradycarde Eigenschaften besitzen.

In der Formel I bedeutet R den n-Propyl-, den Cyclopropylmethyl-, Cyclopentylmethyl-, Allyl- oder Methallylrest.

Obwohl die erfindungsgemäßen Verbindungen denjenigen strukturell sehr nahe kommen, welche aus den oben zitierten Druckschriften bekannt sind, war aus dem Stand der Technik nicht vorherzusehen, daß sie eine bradycarde Wirkung entfalten.

Die Herstellung der Verbindungen der Formel (I) erfolgt durch:

(a) Umsetzung von 2-(2,6-Dibrom-4-methylphenyl-imino)-imidazolidin der Formel (II)

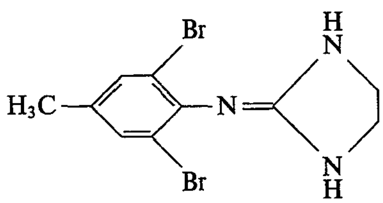

(II)

mit einem Halogenid der allgemeinen Formel (III)

Hal—R (III)

worin Hal ein Chlor-, Brom- oder Jodatom bedeutet und R die oben angeführten Bedeutungen hat; oder

(b) Umsetzung einer Verbindung der allgemeinen Formel (IV)

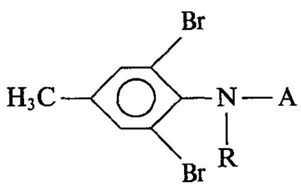

(IV)

in der R wie oben angegeben definiert ist und A eine Cyanogruppe oder den Rest

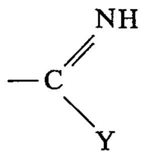

bedeutet, wobei Y eine Alkoxy- oder Alkylthiogruppe mit bis zu 4 C-Atomen oder eine Sulfhydrilgruppe oder eine Aminogruppe darstellt, mit Äthylendiamin oder dessen Säureadditionssalzen.

Die Umsetzung nach Verfahren (a) erfolgt durch Erhitzen der Reaktionspartner — vorzugsweise in Gegenwart eines polaren oder unpolaren Lösungsmittels — auf Temperaturen von 40 bis 150° C.

Die speziellen Reaktionsbedingungen hängen in starkem Maße von der Reaktivität der Umsetzungsteilnehmer ab. Es empfiehlt sich, bei der Alkylierung das Halogenid der allgemeinen Formel (III) im Überschuß anzuwenden und die Umsetzung in Gegenwart eines säurebindenden Mittels durchzuführen.

Beim Verfahren (b) ist es erforderlich, bei erhöhter Temperatur, zwischen 60° und 180°C, zu arbeiten. Lösungsmittel sind nicht notwendig, können aber eingesetzt werden.

Ausgangsverbindungen der Formeln (II) und (IV) sind literaturbekannt (vgl. z. B. BE-A-623 305 oder Chem. Ber. 107, 2644 (1974) oder Liebigs Ann. Chem. 751, 159 (1971)). Verbindungen der Formel (III) lassen sich durch Halogenierung der zugrundeliegenden Alkohole darstellen.

Die erfindungsgemäßen 2-Phenylamino-imidazoline-(2) der allgemeinen Formel (I) können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin.

Die neuen Verbindungen der allgemeinen Formel (I) und deren physiologisch verträgliche Säureadditionssalze wirken sehr stark bradycard und sind daher zur Therapie von Coronarerkrankungen geeignet. Die Beeinflussung der Herzfrequenz wurde an Kaninchen und an Spinalratten sowie intakten, narkotisierten Ratten untersucht.

Die Dosierung liegt bei 0,1 bis 80 mg, vorzugsweise 1 bis 30 mg.

Die Verbindung der Formel (I) bzw. ihre physiologisch verträglichen Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung.

## Herstellungsbeispiele

### Beispiel 1

2-[N-(Cyclopropylmethyl)-N-(2,6-dibrom-4-methyl-phenyl)amino]-2-imidazolin-hydrochlorid

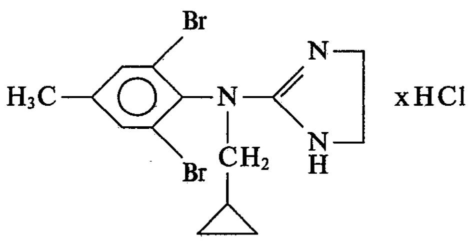

4,2 g (0,013 Mol) 2-[(2,6-Dibrom-4-methyl-phenyl)-imino]-imidazolidin werden zusammen mit 1,7 g (150%) Chlormethylcyclopropan in 25 ml Acetonitril 42 Stunden am Rückfluß unter Rühren erhitzt. Das Reaktionsgemisch wird hierauf im Vakuum zur Trockene eingeengt und der verbleibende Rückstand in etwa 1 N HCl gelöst. Die salzsaure Lösung wird zweimal mit Äther extrahiert (Ätherextrakte werden verworfen) und anschließend bei aufsteigenden pH-Werten (Alkalisieren mit 2 N NaOH) fraktioniert mit Äther extrahiert. Von den erhaltenen 12 Ätherfraktionen werden die einheitlichen (Dünnschichtchromatogramm-Kontrolle) vereinigt, über Magnesiumsulfat getrocknet und mit ätherischer Salzsäure bis zur kongosauren Reaktion versetzt. Hierbei fällt das neue Imidazolinhydrochlorid aus. Es wird abgesaugt, mit Äther gewaschen und getrocknet.

| | |
|---|---|
| Ausbeute: | 1,9 g (entsprechend 36,5% der Theorie). |
| Schmelzpunkt: | 140°C. |

Analog dem vorstehenden Beispiel wurden die folgenden Verbindungen der Formel (I) hergestellt:

| Beispiel Nr. | R | Verfahren | Ausb. (% d. Th.) | Schmp. (°C) |
|---|---|---|---|---|
| 2 | 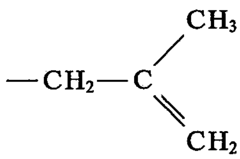 | a | 57,7 | 152–153 (Base) |
| 3 | n-$C_3H_7$ | a | 77,5 | 280 (Hydrobromid) |
| 4 | 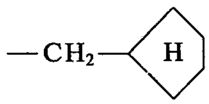 | a | 9,3 | 92–93 (Base) |
| 5 | $-CH_2-CH{=}CH_2$ | a | 80,7 | 272–274 (Hydrobromid) |

## Formulierungsbeispiele

### Beispiel A: Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

### Beispiel B: Ampullen

| | |
|---|---|
| Wirkstoff gemäß Erfindung | 1,0 mg |
| Natriumchlorid | 18,0 mg |
| dest. Wasser ad | 2,0 ml |

Herstellung:

Wirkstoff und Natriumchlorid werden in Wasser gelöst und unter Stickstoff in Glasampullen abgefüllt.

### Beispiel C: Tropfen

| | |
|---|---|
| Wirkstoff gemäß Erfindung | 0,02 g |
| p-Hydroxybenzoesäuremethylester | 0,07 g |
| p-Hydroxybenzoesäurepropylester | 0,03 g |
| entmineralisiertes Wasser ad | 100 ml |

**Patentansprüche (für die Vertragsstaaten BE, CH, DE, FR, GB, IT, LI, LU, NL, SE)**

1. Substituierte 2-Phenylamino-imidazoline-(2) der allgemeinen Formel (I)

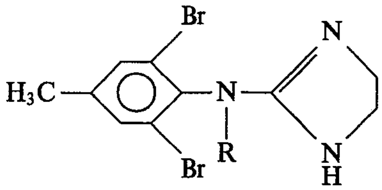

(I)

worin R den n-Propyl-, den Cyclopropylmethyl-, Cyclopentylmethyl-, Allyl- oder Methallylrest bedeutet sowie deren physiologisch verträglichen Säureadditionssalze.

2. Verfahren zur Herstellung von substituierten 2-Phenylamino-imidazolinen gemäß Anspruch 1, dadurch gekennzeichnet, daß man

a) 2-(2,6-Dibrom-4-methylphenyl-imino)-imidazolidin der Formel (II)

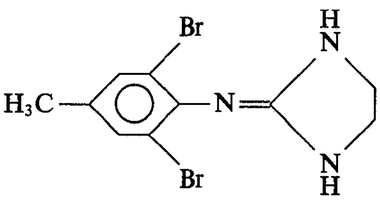

(II)

mit einem Halogenid der allgemeinen Formel (III)

Hal—R (III)

worin Hal ein Chlor-, Brom- oder Jodatom bedeutet und R die oben angeführten Bedeutungen hat bei 40°C bis 150°C umsetzt, oder

(b) eine Verbindung der allgemeinen Formel (IV)

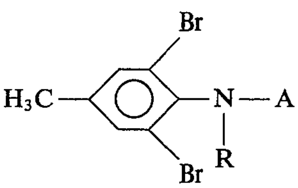

(IV)

in der R wie oben angegeben definiert ist und A eine Cyanogruppe oder den Rest

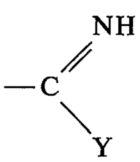

bedeutet, wobei Y eine Alkoxy- oder Alkylthiogruppe mit bis zu 4 C-Atomen oder eine Sulfhydrilgruppe oder eine Aminogruppe darstellt, mit Äthylendiamin oder dessen Säureadditionssalzen bei 60 bis 180° C umsetzt,

und daß man gegebenenfalls das nach einem dieser Verfahren erhaltene Endprodukt in ein entsprechendes Säureadditionssalz überführt.

3. Pharmazeutische Zubereitungen, dadurch gekennzeichnet, daß sie als Wirkstoff eine oder mehrere Verbindungen gemäß Anspruch 1 enthalten.

4. Verfahren zur Herstellung von Arzneimitteln nach Anspruch 3, dadurch gekennzeichnet, daß man eine oder mehrere Verbindungen nach Anspruch 1 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

5. Verbindungen der allgemeinen Formel I oder deren physiologisch verträglichen Säureadditionssalze nach Anspruch 1 zur Verwendung bei der Bekämpfung von Herz- und Coronarerkrankungen.

**Patentansprüche (gültig für den Vertragsstaat AT)**

1. Verfahren zur Herstellung von substituierten 2-Phenylamino-imidazolinen-(2) der allgemeinen Formel (I)

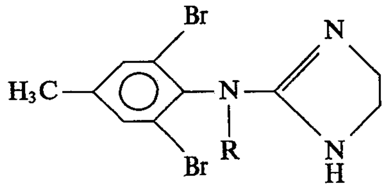

(I)

worin R den n-Propyl-, den Cyclopropylmethyl-, Cyclopentylmethyl-, Allyl- oder Methallylrest bedeutet sowie von deren physiologisch verträglichen Säureadditionssalzen, dadurch gekennzeichnet, daß man

a) 2-(2,6-Dibrom-4-methylphenyl-imino)-imidazolidin der Formel (II)

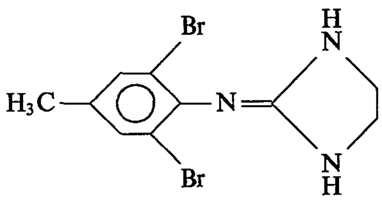

(II)

mit einem Halogenid der allgemeinen Formel (III)

Hal—R (III)

worin Hal ein Chlor-, Brom- oder Jodatom bedeutet und R die oben angeführten Bedeutungen hat bei 40°C bis 150°C umsetzt, oder

b) eine Verbindung der allgemeinen Formel (IV)

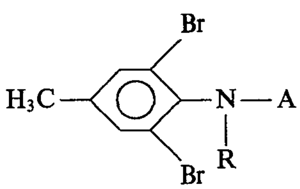

(IV)

in der R wie oben angegeben definiert ist und A eine Cyanogruppe oder den Rest

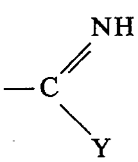

bedeutet, wobei Y eine Alkoxy- oder Alkylthiogruppe mit bis zu 4 C-Atomen oder eine Sulfhydrilgruppe oder eine Aminogruppe darstellt, mit Äthylendiamin oder dessen Säureadditionssalzen bei 60 bis 180° C umsetzt,

und daß man gegebenenfalls das nach einem dieser Verfahren erhaltene Endprodukt in ein entsprechendes Säureadditionssalz überführt.

2. Verfahren zur Herstellung von Arzneimitteln, dadurch gekennzeichnet, daß man eine oder mehrere Verbindungen gemäß Anspruch 1 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

**Claims for the Contracting states: BE, CH, DE, FR, GB, IT, LI, LU, NL, SE**

1. Substituted 2-phenylamino-imidazolines-(2) of general formula (I)

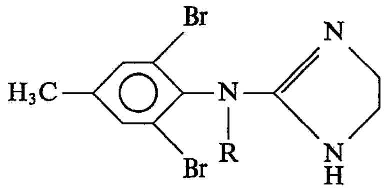

(I)

wherein R represents an n-propyl, cyclopropylmethyl, cyclopentylmethyl, allyl or methallyl group, and the physiologically acceptable acid addition salts thereof.

2. Process for preparing substituted 2-phenylamino-imidazolines as claimed in claim 1, characterised in that

a) 2-(2,6-dibromo-4-methylphenyl-imino)-imidazolidine of formula (II)

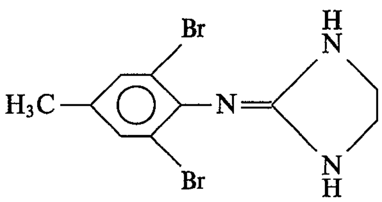

(II)

is reacted with a halide of general formula (III)

Hal—R (III)

wherein Hal represents a chlorine, bromine or iodine atom and R is as hereinbefore defined, at 40°C to 150°C, or

b) a compound of general formula IV

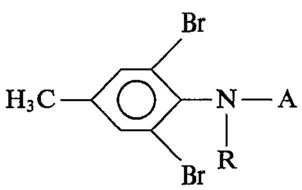

(IV)

wherein R is as hereinbefore defined and A represents a cyano group or the group

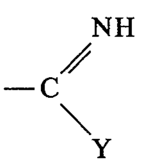

wherein Y represents an alkoxy or alkylthio group with up to 4 carbon atoms or a mercapto group or an amino group, is reacted with ethylenediamine or the acid addition salts thereof at 60 to 180° C,

and the end product obtained according to one of these methods is optionally converted into a corresponding acid addition salt.

3. Pharmaceutical preparations, characterised in that they contain, as active substance, one or more compounds as claimed in claim 1.

4. Process for preparing pharmaceutical compositions as claimed in claim 3, characterised in that one or more compounds as claimed in claim 1 are combined with conventional galenic excipients, carriers, disintegrants or lubricants or substances for obtaining delayed release, to form tablets, capsules, suppositories, solutions or powders.

5. Compounds of general formula I or the physiologically acceptable acid addition salts thereof as claimed in claim 1 for use in combatting cardiac and coronary diseases.

**Claims for the Contracting state AT**

1. Process for preparing substituted 2-phenylamino-imidazolines-(2) of general formula (I)

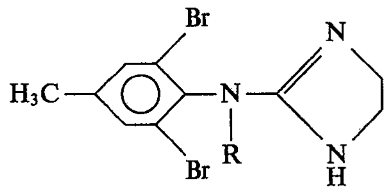

(I)

wherein R represents an n-propyl, cyclopropylmethyl, cyclopentylmethyl, allyl or methallyl group, and the physiologically acceptable acid addition salts thereof, characterised in that

a) 2-(2,6-dibromo-4-methylphenyl-imino)-imidazolidine of formula II

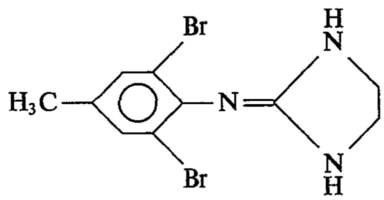

(II)

is reacted with a halide of general formula III

$$Hal—R \qquad (III)$$

wherein Hal represents a chlorine, bromine or iodine atom and R is as hereinbefore defined, at 40°C to 150°C, or

b) a compound of general formula IV

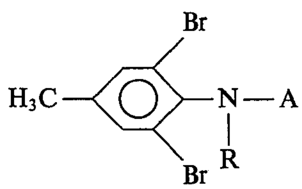

(IV)

wherein R is as hereinbefore defined and A represents a cyano group or the group

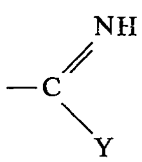

wherein Y represents an alkoxy or alkylthio group with up to 4 carbon atoms or a mercapto group or an amino group, is reacted with ethylenediamine or the acid addition salts thereof at 60 to 180° C,

and the end product obtained according to one of these processes is optionally converted into a corresponding acid addition salt.

2. Process for preparing pharmaceutical compositions, characterised in that one or more compounds as claimed in claim 1 is or are combined with conventional galenic excipients, carriers, disintegrants or lubricants or substances for obtaining delayed release, to produce tablets, capsules, suppositories, solutions or powders.

**Revendications pour les Etats contractants: BE, CH, DE, FR, GB, IT, LI, LU, NL, SE**

1. 2-phénylamino-imidazolines-(2) substituées de formule générale (I)

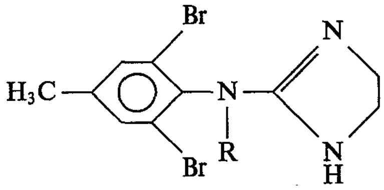

(I)

dans laquelle R représente le radical n-propyle, cyclopropylméthyle, cyclopentylméthyle, allyle ou méthallyle ainsi que leurs sels d'addition d'acides physiologiquement supportables.

2. Procédé pour la préparation de 2-phénylamino-imidazolines substituées selon la revendication 1, caractérisé en ce que

(a) on fait réagir 2-(2,6-dibromo-4-méthylphenyl-imino)-imidazolidine de formule (II)

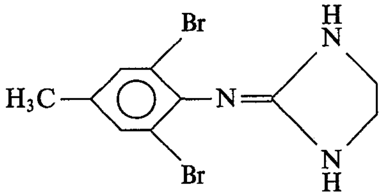

(II)

avec une halogénure de formule générale (III)

Hal—R (III)

dans laquelle Hal représente un atome de chlore, de brome ou d'iode et R a les significations indiquées plus haut à 40°C à 150°C, ou

b) on fait réagir un composé de formule générale (IV)

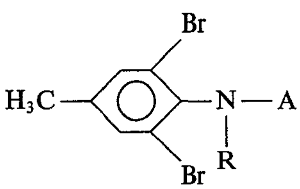

(IV)

dans laquelle R est défini comme indiqué plus haut et A représente un groupe cyano ou le radical

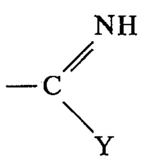

Y représentant un groupe alcoxy ou alcoylthio avec jusqu'à 4 atomes de C ou un groupe sulfhydryle ou un groupe amino, avec l'éthylènediamine ou ses sels d'addition d'acides à 60 à 180° C,

et en ce qu'éventuellement on transforme le produit final obtenu selon l'un de ces procédés en un sel d'addition d'acide correspondant.

3. Préparations pharmaceutiques, caractérisées en ce qu'elles contiennent en tant que substance active un ou plusieurs composés selon la revendication 1.

4. Procédé pour la préparation de médicaments selon la revendication 3, caractérisé en ce qu'on formule un ou plusieurs composés selon la revendication 1 avec des adjuvants, excipients, désagrégeants ou lubrifiants ou respectivement substances pour obtenir un effet retard habituels, en comprimés, capsules, suppositoires, solutions ou poudres.

5. Composés de formule générale I ou leurs sels d'addition d'acides physiologiquement supportables selon la revendication 1 pour l'utilisation dans le combat contre les maladies du coeur et les maladies des coronaires.